# EUROPÄISCHE PATENTANMELDUNG

(11) **EP 1 882 471 A2**
(43) Veröffentlichungstag der Anmeldung: **30.01.2008**
(21) Anmeldenummer: 07110723.9
(22) Anmeldetag: 21.06.2007
(51) Int. Cl.: A61K 8/49, A61Q 19/08, A61K 8/67

(54) **Kosmetische Zubereitung umfassend Folsäure und Parfümbestandteile**

(30) Priorität: 28.07.2006 DE 102006035789
(71) Anmelder: Beiersdorf AG, 20253 Hamburg (DE)
(72) Erfinder: von der Fecht, Stephanie, 22869, Schenefeld (DE); Kröpke, Rainer, 22869, Schenefeld (DE); Zilz, Werner, 25469, Halstenbek (DE); Treu Dr., Jens, 22844, Norderstedt (DE); Schulz Dr., Jens, 22869, Schenefeld (DE); Drucks, Anja, 22399, Hamburg (DE)

(57) **Zusammenfassung**

Die vorliegende Erfindung betrifft kosmetische Zubereitungen auf der Grundlage von Folsäure umfassend bestimmte Parfumbestandteile. Die Kombinationen führt zur Verbesserung der olfaktorische Eigenschaft Folsäure-haltiger und/oder deren Derivate enthaltende kosmetische und/oder dermatologische Zubereitungen. Ebenso wird eine Stabilisierung von Folsäure und/oder deren Derivate durch die bestimmten Parfumbestandteile in kosmetischen und/oder dermatologischen Zubereitungen beobachtet.

## Beschreibung

Die vorliegende Erfindung betrifft kosmetische Zubereitungen auf der Grundlage von Folsäure umfassend Parfumbestandteile.

Die vorliegende Erfindung betrifft in bevorzugten Ausführungsformen kosmetische bzw. dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Die menschliche Haut übt als größtes Organ des Menschen zahlreiche lebenswichtige Funktionen aus. Mit durchschnittlich etwa 2 m² Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Au-ßerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Hornschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die kosmetische und dermatologische Verwendung von Folsäure ist an sich bekannt. So beschreibt die DE 100 62 401 die Verwendung von Folsäure und/oder deren Derivaten zur Herstellung kosmetischer oder dermatologischer Zubereitungen zur Prophylaxe von Schäden an der hauteigenen DNA und/oder zur Reparatur bereits eingetretener Schäden an der hauteigenen DNA.

Folsäure weist folgende Struktur auf:

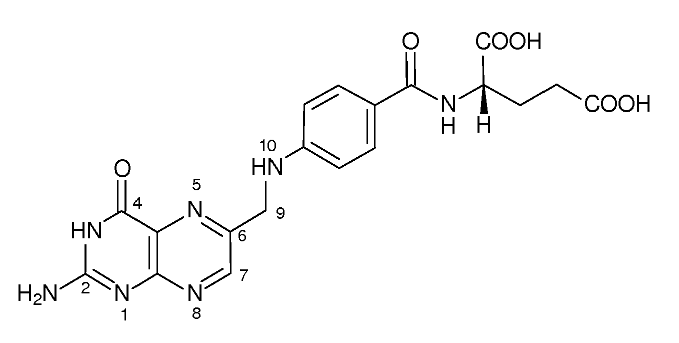

Folsäure kommt in Leber, Niere, Hefe, Pilzen, Getreide und grünen Blättern, vorwiegend als Konjugat mit Poly- -L-glutaminsäure (Pteroylpolyglutaminsäuren) vor. Sie wurde als Wuchsstoff für verschiedene Mikroorganismen entdeckt und hat für den menschlichen Organismus Vitamincharakter. Der Bedarf des erwachsenen Menschen beträgt etwa 200 µg je Tag an bioverfügbarem Folat.

Folsäure steht im Organismus im Gleichgewicht mit 7,8-Dihydrofolsäure (H₂Folat; alte Abkürzung: FH₂) unter Beteiligung von Nicotinamid-Adenin-Dinucleotid-Phosphat und des Enzyms Dihydrofolat-Reduktase. H₂Folat wiederum entsteht in Pflanzen und einigen Mikroorganismen über mehrere Zwischenstufen aus Guanosin-5'-triphosphat (Guanosinphosphate) und setzt sich mit Hilfe der Dihydrofolat-Reduktase zu (6S)-5,6,7,8-Tetrahydrofolsäure um, der eigentlichen physiologisch wirksamen Form der Folsäure.

7,8-Dihydrofolsäure hat folgende chemische Struktur:

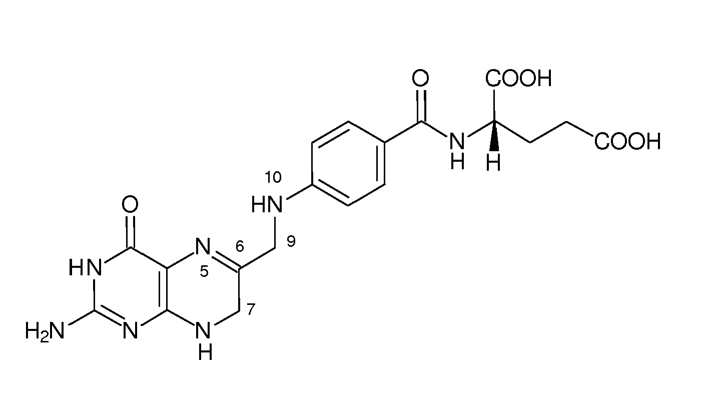

(6S)-5, 6,7,8-Tetrahydrofolsäure hat folgende chemische Struktur:

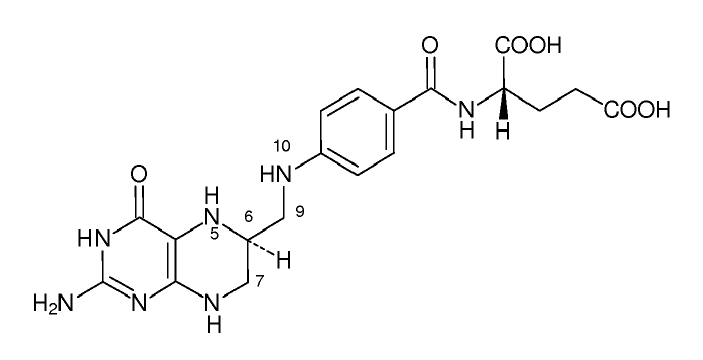

Unter dem Begriff der Derivate der Folsäure sind erfindungsgemäß insbesondere die vorgenannte Dihydrofolsäure und die Tetrahydrofolsäure zu verstehen.

Nachteilig ist bislang, dass Folsäure und ihre Derivate und insbesondere Folsäure-haltige Hautpflegeprodukte einen Nebengeruch ausbilden, der für den Anwender als unangenehm empfunden wird.

Diesen Nebengeruch durch Parfum oder sonstige leichtflüchtige Stoffe mit einem markanten Eigengeruch zu überdecken ist aufgrund der fehlenden Langzeitstabilität der Überdeckung bislang fehlgeschlagen.

Darüber hinaus stellt die Destabilisierung von Wirkstoffen durch Parfumbestandteile ein Problem bei Entwicklung kosmetischer Zubereitungen dar.

Diesem Nachteil des Standes der Technik galt es also abzuhelfen.

Überraschenderweise zeichnen sich kosmetische Zubereitungen mit einem Gehalt an Folsäure und/oder deren Derivaten sowie mindestens einem Parfumbestandteil gewählt aus der Gruppe
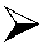 2-Isobutyl-4-hydroxy-4-methyltetrahydropyran
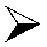 2-tert-Pentylcyclohexylacetat
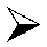 3-Methyl-5-phenyl-1-pentanol
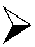 7-Acetyl-1,1,3,4,4,6-hexamethyltetralin
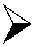 Adipinsäurediester
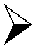 alpha-Amylcinnamaldehyd
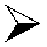 alpha-Isomethylionon
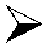 Alpha-Methylionon
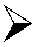 Amyl C Butylphenyl,ethylpropionalcinnamal
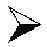 Amylsalicylat
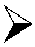 Amylcinnamylalkohol
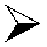 Anisalkohol
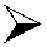 Benzoin
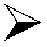 Benzylalkohol
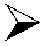 Benzylbenzoat
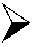 Benzylcinnamat
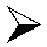 Benzylsalicylat
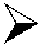 Bergamotöl
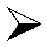 bitteres Orangenöl
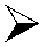 Butylphenylmethylpropioal
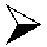 Cardamomöl
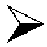 Cedrol
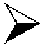 Cinnamal
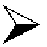 Cinnamylalkohol
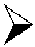 Citral
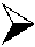 Citronelol
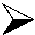 Citronellylmethylcrotonat
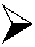 Citronenöl
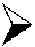 Coumarin
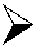 Diethylsuccinat
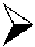 d-Limonene
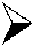 Ethyllinalool
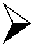 Eugenol
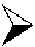 Evernia Furfuracea Extract
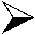 Evernia Prunastri Extract
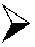 Farnesol
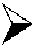 Geraniol
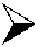 Guajakholzöl
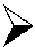 Hexylcinnamal
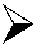 Hexylsalicylat
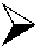 Hydroxycitronellal
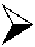 Hydroxyisohexyl 3-Cyclohexencarboxaldehyde
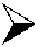 Isoeugenol
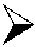 Lavendelöl
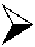 Lemonenöl
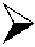 Limonen
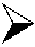 Linalool
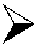 Linaylacetat
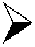 Mandarinenöl
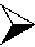 Menthyl PCA
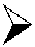 Methyl 2-Octynoat
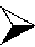 Methylbenzoat
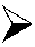 Methylheptenon
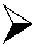 Muskatnussöl
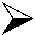 Rosmarinöl
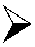 süßes Orangenöl
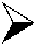 Terpineol
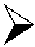 Tonkabohnenöl
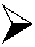 Triethylcitrat und/oder
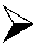 Vanillin
durch verbesserte olfaktorische Eigenschaften aus und überkommen erfindungsgemäß die Nachteile des Standes der Technik.

Als besonders bevorzugte Parfumbestandteile können Methylbenzoat, Limonen, Citral, Linalool, alpha-Isomethylionone, Geraniol, Methyl-2 Octynoat, Citronelol, Isoeugenol, Benzylbenzoat, Benzylalkohol und/oder Benzylsalicylat gewählt werden.

Bevorzugt sind als Parfumbestandteile Methylbenzoat, Citral, Limonen, Benzylbenzoat, Isoeugenol und/oder Citronelol auszuwählen.

Vorteilhaft hat sich gezeigt, dass insbesondere die Kombination von zwei, drei, vier oder fünf der bestimmten Parfumbestandteile zu einer verbesserten olfaktorischen Wirkung der Folsäurehaltigen Zubereitung führt.

So ist eine Zweier-Kombination, beispielsweise Methylbenzoat und Citral, Isoeugenol und Citronelol oder Isoeugenol und Benzylbenzoat, bevorzugt zu wählen.

Auch eine Dreier-Kombination, wie beispielsweise Citral, alpha-Isomethylionone und Benzylsalicylat ist eine vorteilhafte Wahl.

Bei manchen Folsäurehaltigen Zubereitungen des Standes der Technik, die keine oder andere als die erfindungsgemäß ausgewählten Parfumbestandteile umfassen, stellt sich der unangenehme Nebengeruch manchmal erst nach einigen Tagen oder Wochen ein. Damit ist die Herstellung Folsäurehaltiger Kosmetika problematisch, da sich ggf. erst bei Anwendung beim Kunden der Nachteil des unangenehmen Geruchses bermekbar macht.

Deshalb muss der Parfumbestandteil bzw. die Kombination der Bestandteile und Kombination mit dem entsprechenden Wirkstoff sehr sorgfältig ausgewählt werden.

Die erfindungsgemäßen Zubereitungen weisen überraschenderweise auch in einem Crashtest bei 40 °C über 6 Monate einen angenehmen Geruch auf.

In der Parfumprüfung werden dazu im Zeitabstand von 2, 4 und 6 Monaten die gelagerten Proben bei +25°C und +40°C und Lichtmuster im Vergleich zu einem bei +6°C gelagertem Muster von einen Parfumeur begutachtet.

### Ergebnisse der Parfumprüfung nach 6 Monaten Testzeit:

| Rezeptur | Beispiel 1 ohne Parfumbestandteil | Beispiel 1 mit Parfumbestandteil | Beispiel 2 ohne Parfumbestandteil | Beispiel 2 mit Parfumbestandteil |
|---|---|---|---|---|
| +6°C-Muster | Typischer Rohstoffgeruch | In Ordnung | Typischer Rohstoffgeruch | In Ordnung |
| +25°C-Muster | Technisch, sauer | In Ordnung | Technisch, stechend, leicht süßlich | In Ordnung |
| +40°C-Muster | Technisch, muffig | Nur leichter Frischeverlust | Technisch, stechend, sauer | In Ordnung |
| Licht-Muster | Stechend-sauer | Nur leichter Frischeverlust | Stechend-sauer | Nur leichter Frischeverlust |

Der Anteil an einem oder mehreren Parfumbestandteilen beträgt vorteilhaft mindestens 0,00001 Gew.-%, bevorzugt mindestens 0,001 Gew.-%, bezogen auf die Gesamtmasse der Zubereitung. Maximal ist ein Anteil von 1 Gew.-%, bevorzugt maximal 0,4 Gew.-%, bezogen auf die Gesamtmasse der Zubereitung, vorteilhaft.

Es ist vorteilhaft, die Gesamtmenge an Parfumbestandteilen, eine oder mehrere der genannten Verbindungen, zu einem Anteil von 0,00001 bis 1 Gew.-%, bevorzugt von 0,001 bis 0,4 Gew.-%, bezogen auf die Gesamtmasse der Zubereitung, zu wählen.

Überraschend hat sich auch gezeigt, das die genannten Parfumbestandteile allein sowie bevorzugt in bestimmten Mischungen (zweier-, Dreier-, Vierer oder Fünfer-Mischungen), die Folsäure stabilisieren können.

Sie enthalten bevorzugt 0,001 Gew.-% bis 0,5 Gew.-%, besonders bevorzugt 0,005 Gew.-% bis 0,2 Gew.-%, insbesondere 0,01 bis 0,1 Gew.-%, bezogen auf das Gesamtgewicht der Zubereitungen, an Folsäure und/oder deren Derivaten.

Die kosmetischen oder dermatologischen Zubereitungen können erfindungsgemäß wie üblich zusammen gesetzt sein und zur Behandlung, der Pflege und der Reinigung der Haut und/oder der Haare und als Schminkprodukt in der dekorativen Kosmetik dienen.

Die erfindungsgemäßen Zubereitungen können weiterhin bevorzugt Polyole umfassen. Dies insbesondere, wenn die Zubereitungen eine gewisse pH-Wert Unabhängigkeit aufweisen sollen.

Vorteilhafte Beispiele für Poylyole sind: Erythrit, Arabit, Adonit, Sorbit, Dulcit, 1,2-Butandiol, 1,3-Butandiol, 1,4-Butandiol, 2,3-Butandiol, 1,5-Pentandiol, 2,4-Pentandiol, 2-Methyl-1,3-Propandiol, 2-Methyl-2,4-pentandiol, 1,6-Hexandiol, 2,3-Dimethyl-2,3-butandiol, 2,2-Diethyl-1,3-propandiol, 2-Ethyl-1,3-hexandiol, 2,2,4-Trimethyl-1,3-pentandiol, 2-Ethyl-2-butyl-1,3-propandiol, 1,2,4-Butantriol, 1,2,6-Hexantriol, 2,2-Dihydroxymethyl-1-butanol, 1,2-Octandiol,Tetramethylolmethan, dem -Mono-methylether von Glycerin, dem -Mono-n-butylether von Glycerin, 2-O- -D-Glucopyranosyl-L-ascorbinsäure, 3-(2-Ethylhexyloxy)-1,2-propandiol, 1,2-Hexandiol.

Bevorzugt sind Polyole, die 2 bis 3 Sauerstoffatome enthalten und mindestens 4 Kohlenstoffatome.

Das Verhältnis von Folsäure zu Polyol (bzw. der Gesamtmenge an Polyolen) sollte 1:10 bis 1:1000 betragen.

Die kosmetischen oder dermatologischen Zubereitungen enthalten ferner bevorzugt 0,001 Gew.-% bis 30 Gew.-%, bevorzugt 0,05 Gew.-% bis 10 Gew.-%, insbesondere 0,1 - 2,0 Gew.-%, bezogen auf das Gesamtgewicht der Zubereitungen, an einem oder mehreren Polyolen.

Eine kosmetische Zubereitung umfassend Folsäure und/oder deren Derivaten, mindestens ein Parfumbestandteil gewählt aus der Gruppe 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, alpha-Isomethylionon, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citral, Citronellol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Geraniol, Guajakholzöl, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linaylacetat, Mandarinenöl, Menthyl PCA, Methyl 2-Octynoat, Methylbenzoat, Methylheptenon, Muskatnussöl, Rosmarinöl, süßes Orangenöl, Terpineol, Tonkabohnenöl, Triethylcitrat und/oder Vanillin,
sowie insbesondere Methylbenzoat, Limonene, Citral, Linalool, alpha-Isomethylionone, Geraniol, Methyl-2 Octynoat, Citronellol, Isoeugenol, Benzylbenzoat, Benzylalkohol und/oder Benzylsalicylat und insbesondere 0,1 - 2,0 Gew.-%, bezogen auf das Gesamtgewicht der Zubereitungen, an einem oder mehreren Polyolen, bildet eine bevorzugte kosmetische Zubereitung.

Die erfindungsgemäßen Zubereitungen können weiterhin bevorzugt Füllstoffe und Verdicker umfassen.

Erfindungsgemäße Folsäurehaltige Hautpflegeprodukte sollten neben dem positiven Effekt auf die Haut auch als angenehm anzuwenden sein. Puderrohstoffe geben ein angenehmes seidig samtes Hautgefühl. Allerdings destabilisieren sowohl Folsäure (Elektrolyt) als auch Puderrohstoffe eine Emulsion, so dass die Kombination in einer Emulsion häufig nicht lager- und wärmestabil ist.

Aus diesem Grund ist es bevorzugt den Zubereitungen Füllstoffe und/oder Verdicker zuzusetzen.

Füllstoffe im Sinne der vorliegenden Erfindung sind partikuläre Substanzen, die in der Regel keinen Farbeffekt in der kosmetischen Formulierung erzeugen, in der sie eingesetzt werden. Ferner haben erfindungsgemäße Füllstoffe üblicherweise einen niedrigen Brechungsindex und daraus resultierend keine oder eine nur sehr geringe Deckkraft.

Der Stand der Technik kennt eine Reihe von Füllstoffen, welche z. B. als Trägermaterialien bei der Formulierung von Pudern oder als Viskositäts- und Sensorik-Modulatoren in Emulsionen oder wasserfreien Formulierungen dienen. Derartige Füllstoffe werden häufig auch eingesetzt, um mattierende Effekte auf der Haut zu erlangen oder um Sebum zu absorbieren.

Darüber hinaus beeinflusst der Einsatz von Füllstoffen im Allgemeinen auch die Verteilbarkeit üblicher Formulierungen auf der Haut sowie die Gleichmäßigkeit eines möglichen Farbeffektes.

Die Füllstoffe im Sinne der vorliegenden Erfindung werden vorteilhaft aus der Gruppe der anorganische Füllstoffe gewählt, beispielsweise aus der Gruppe der Silikate.

Silikate sind Salze und Ester (Kieselsäureester) der Orthokieselsäure [Si(OH)₄] und deren Kondensationsprodukte. Die Silikate sind nicht nur die artenreichste Klasse der Mineralien, sondern auch geologisch und technisch außerordentlich wichtig. Über 80 % der Erdkruste bestehen aus Silikaten.

Vorteilhaft im Sinne der vorliegenden Erfindung sind beispielsweise Schichtsilikate. Schichtsilikate (Phyllosilikate, Blattsilikate) sind (idealerweise) Silikat-Strukturen mit zweidimensional unendlichen Schichten aus [SiO₄]⁴⁻-Tetraedern, wobei jedes Tetraeder über 3 Brücken-Sauerstoffe mit Nachbar-Tetraedern verbunden ist.

Chemische Formeln lassen sich für Schichtsilikate nur angenähert aufstellen, da sie ein großes lonenaustausch-Vermögen besitzen und Silicium gegen Aluminium und dieses wiederum gegen Magnesium, Fe²⁺, Fe³⁺, Zn und dergleichen ausgetauscht werden kann. Die daraus möglicherweise resultierende negative Ladung der Schichten wird in der Regel durch Kationen, insbesondere durch Na⁺ und Ca²⁺ in Zwischenschicht-Positionen ausgeglichen.

Schichtsilikate können durch reversible Einlagerung von Wasser (in der 2- bis 7-fachen Menge) und anderen Substanzen wie z. B. Alkoholen, Glykolen und dergleichen mehr aufquellen. Ihre Verwendung als Verdickungsmittel in kosmetischen Mitteln ist dementsprechend an sich bekannt. Allerdings konnte der Stand der Technik nicht den Weg zur vorliegenden Erfindung weisen.

Vorteilhafte Schichtsilikate, welche im Sinne der vorliegenden Erfindung eingesetzt werden können, sind beispielsweise solche, deren größte Ausdehnungsrichtung im unmodifizierten und ungequollenen Zustand im Mittel eine Länge von weniger als 10 µm hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung hängt selbstverständlich von der Menge an eingelagerten Substanzen ab.

Erfindungsgemäß vorteilhafte (Schicht-) Silikate sind insbesondere:
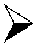 Talkum: Mg₃ [Si₄O₁₀] (OH)₂,
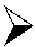 Kaolin: Al₂[Si₂O₅] (OH)₄
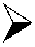 Montmorillonit: M⁺ Al [Si₂O₅](OH), auch Smektite genannt. Darunter fallen:
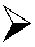 Bentonite = Montmorillonite mit Ca (Fuller Erden) oder Na (Wyoming Bentonite)
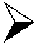 Hektorite: M⁺_{0,3}(Mg_{2,7}Li_{0,3})[Si₄O₁₀(OH)₂], worin M⁺ meist Na⁺ darstellt,
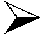 Glimmer (Mica), ein Alumosilikat, das leicht spaltbar ist und in tafeligen Kristallen vorliegt. Glimmer ist transparent bis durchscheinend und weist Perlglanz auf. Die wichtigste Form ist Muskovit: K Al₂ [AlSi₃O₁₀] (OH, F)₂. Sericite ist eine Sonderform des Glimmers, die kleinere Plättchen als Muskovit aufweist.
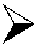 Magnesiumsilikat Mg₂ [Si₄O₁₀]

Auch Siliciumoxide (SiO₂) sind vorteilhaft im Sinne der vorliegenden Erfindung zu verwenden. Erfindungsgemäß bevorzugt sind beispielsweise Aerosile (fumed Silica), welche hochdisperse Kieselsäuren mit häufig irregulärer Form sind, deren spezifische Oberfläche in der Regel sehr groß ist (200 - 400 m²/ g) und mit Hilfe des Herstellverfahrens gesteuert werden kann. Aerosile werden auch bezeichnet als: Amorphous Silica Amorphous Silicon Oxide Hydrate Silica, Amorphous Silicic Anhydride Silicon Dioxide Silicon Dioxide.

Erfindungsgemäß vorteilhafte Aerosile sind z. B. unter den folgenden Handelsnamen erhältlich: Aerosil 130 (Degussa Hüls), Aerosil 200 (Degussa Hüls), Aerosil 255 (Degussa Hüls), Aerosil 300 (Degussa Hüls), Aerosil 380 (Degussa Hüls), B-6C (Suzuki Yushi), CAB-O-SIL Fumed Silica (Cabot), CAB-O-SIL EH-5 (Cabot), CAB-O-SIL HS-5 (Cabot), CAB-O-SIL LM-130 (Cabot), CAB-O-SIL MS-55 (Cabot), CAB-O-SIL M-5 (Cabot), E-6C (Suzuki Yushi), Fossil Flour MBK (MBK), MSS-500 (Kobo), Neosil CT 11 (Crosfield Co.), Ronasphere (Rona/EM Industries), Silica, Anhydrous 31 (Whittaker, Clark & Daniels), Silica, Crystalline 216 (Whittaker, Clark & Daniels), Silotrat-1 (Vevy), Sorbosil AC33 (Crosfield Co.), Sorbosil AC 35 (Crosfield Co.), Sorbosil AC 37 (Crosfield Co.), Sorbosil AC 39 (Crosfield Co.), Sorbosil AC77 (Crosfield Co.), Sorbosil TC 15 (Crosfield Co.), Spherica (lkeda), Spheriglass (Potters-Ballotini), Spheron L-1500 (Presperse), Spheron N-2000 (Presperse), Spheron P-1500 (Presperse), Wacker HDK H 30 (Wacker-Chemie), Wacker HDK N 20 (Wacker-Chemie), Wacker HDK P 100 H (Wacker Silicones), Wacker HDK N 20P (Wacker-Chemie), Wacker HDK N 25P (Wacker-Chemie), Wacker HDK S 13 (Wacker-Chemie), Wacker HDK T 30 (Wacker-Chemie), Wacker HDK V 15 (Wacker-Chemie), Wacker HDK V 15 P (Wacker-Chemie), Zelec Sil (DuPont).

Siliciumoxide lassen sich auch in sphärischer Form herstellen, wobei hier die spezifische Oberfläche kleiner ist als bei den Aerosilen, da die Teilchen größer und rund sind. Ein Beispiel hierfür sind die Ronaspheren (mittlerer Teilchendurchmesser < 3) der Fa. Merck (siehe Fig 1).

Weitere erfindungsgemäß bevorzugte Füllstoffe sind Siliciumdioxide, deren freien OH-Gruppen an der Teilchenoberfläche (ganz oder teilweise) organisch modifiziert sind.

Vorteilhaft sind z. B. die durch Addition von Dimethylsilyl-Gruppen erhältlichen Silica Dimethyl Silylate, wie beispielsweise Aerosil R972 (Degussa Hüls), Aerosil R974 (Degussa Hüls), CAB-O-SIL TS-610 (Cabot), CAB-O-SIL TS-720 (Cabot), Wacker HDK H15 (Wacker-Chemie), Wacker HDK H18 (Wacker-Chemie) und/oder Wacker HDK H20 (Wacker-Chemie).

Ferner vorteilhaft sind die durch Addition von Trimethylsilylgruppen erhältlichen Silica Silylate (z. B. Aerosil R 812 (Degussa Hüls), CAB-O-SIL TS-530 (Cabot), Sipernat D 17 (Degussa Hüls), Wacker HDK H2000 (Wacker-Chemie)).

Ferner vorteilhaft im Sinne der vorliegenden Erfindung sind die durch Hydrolyse- und Kondensationsreaktionen von Methyltrimethoxysilane erhältlichen Polymethylsilsesquioxane, die ebenfalls eine runde Form besitzen und deren Teilchengrößenverteilung durch die Herstellung gesteuert werden kann.

Bevorzugte Polymethylsilsesquioxane werden beispielsweise unter den Handelsnamen Tospearl 2000 B von GE Bayer Silikones, Tospearl 145A von Toshiba, AEC Silicone Resin Spheres von A & E Connock sowie Wacker - Belsil PMS MK von der Wacker-Chemie angeboten.

Weiterer vorteilhafter Füllstoff im Sinne der vorliegenden Erfindung ist Bornitrid. Bornitrid ist isoelektronisch mit Kohlenstoff (d. h. es sind Graphit- und Diamantform möglich). Bornitrid zeichnet sich durch seine chemische Inertheit aus.

Vorteilhaft im Sinne der vorliegenden Erfindung sind beispielsweise die im folgenden aufgelisteten Bornitride:

| | |
|---|---|
| Handelsname | erhältlich bei: |
| Boron Nitride Powder | Advanced Ceramics |
| Boron Nitride Powder | Sintec Keramik |
| Ceram Blanche | Kawasaki |
| HCST Boron Nitride | Stark |
| Tres BN^{®} | Carborundum |
| Wacker-Bornitrid BNP | Wacker-Chemie |

Weitere vorteilhafte Füllstoffe im Sinne der vorliegenden Erfindung sind Carbonate, wie z. B. Magnesiumcarbonat (MgCO₃) und Calciumcarbonat (CaCO₃). Es ist insbesondere vorteilhaft im Sinne der vorliegenden Erfindung, die Carbonate als Füllstoffe in trockenen Pudern zu verwenden.

Die Füllstoffe im Sinne der vorliegenden Erfindung werden darüber hinaus vorteilhaft aus der Gruppe der organische Füllstoffe gewählt.

Erfindungsgemäß vorteilhafte organische Füllstoffe sind z. B. natürliche Polymere, wie Seidenpuder, mikrokristalline Cellulose und/oder Zinkstearate.

Vorteilhafte organische Füllstoffe sind ferner Stärke und Stärkederivate, wie:
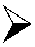 Maisstärke Zea Mays (Amidon De Mais MST (Wackherr), Argo Brand Corn Starch (Corn Products), Pure-Dent (Grain Processing), Purity 21C (National Starch)),
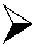 Reisstärke (D.S.A. 7 (Agrana Stärke), Oryzapearl (Ichimaru Pharcos)),
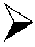 Distarch Phosphate (Corn PO4 (Agrana Stärke), Corn PO4 (Tri-K)),
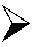 Sodium Corn Starch Octenylsuccinate (C* EmCap - Instant 12639 (Cerestar USA)),
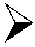 Aluminium Starch Octenylsuccinate (Covafluid AMD (Wackherr), Dry Flo-PC (National Starch), Dry Flo Pure (National Starch), Fluidamid DF 12 (Roquette)),

Erfindungsgemäß bevorzugte organische Füllstoffe sind auch synthetische Polymere, d. h. Polymerpartikel, welche in der Zubereitung in Form von Feststoffen vorliegen, wie beispielsweise Polycarbonate, Polyether, Polyethylen, Polypropylen, Polyvinylchlorid, Polystyrol, Polyamide, Polyurethane, Polyacrylate und dergleichen mehr. Besonders vorteilhaft ist z. B. die Substanz mit der INCI-Bezeichnung *HDI*/*Trimethylol Hexyllactone Crosspolymer,* welche unter der Bezeichnung BPD-500/Plastic Powder D von der Firma Kobo erhältlich ist.

Ferner vorteilhaft im Sinne der vorliegenden Erfindung ist Nylon (Polyamid 6 und Polyamid 12), wie beispielsweise mikrofeine Polyamid-Partikel, insbesondere die unter der Handelsbezeichnung SP-500 bei der Firma TORAY erhältlichen. Ferner vorteilhaft sind Polyamid 6- (auch: Nylon 6), bzw. Polyamid 12- (auch: Nylon 12), Partikel. Polyamid 6 ist das aus ε-Aminocapronsäure (6-Aminohexansäure), oder ε-Caprolactam aufgebaute Polyamid [Poly(ε-caprolactam)], und Polyamid 12 ist ein Poly(ε-laurinlactam), aus ε-Laurinlactam. Vorteilhaft im Sinne der vorliegenden Erfindung sind beispielsweise Orgasol^{®} 1002 (Polyamid 6), und Orgasol^{®} 2002 (Polyamid 12), von der Firma ELF ATOCHEM.

Weitere vorteilhafte organische Füllstoffe sind:
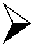 PMMA: Polymethylmethacrylate
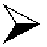 Polyethylene Spheres
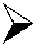 Polyurethane
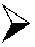 Silikon Resins: Trimethylsiloxysilicate (z. B. SR 1000 GE Bayer Silicones)
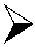 Silikonelastomere

So kann es z. B. von erheblichem Vorteil sein, solche Silikonelastomeren in Zubereitungen gemäß der vorliegenden Erfindung zu verwenden, wie sie beispielsweise in den US-Patenten US 4980167 oder US 4742142 beschrieben werden. Vorteilhafte Silikonelastomere sind ferner z. B. solche, welche unter den Namen KSG6 von Shin Etsu, Tefil E-505C oder Trefil E-506C von Dow Corning, Gransil von Grant Industries (SR-CYC, SR-DMF10, SR-DC556), vertrieben werden sowie solche, die in Form von vorgefertigten Gelen verkauft werden (wie z. B. KSG15, KSG17, KSG16, KSG18 von Shin Etsu, Gransil SP 5CYC Gel, Gransil SR DMF 10 Gel, Gransil SR DC 556 Gel, Gransil GCM, Gransil PM Gel, Gransil DMG-5, SF 1204 und JK 113 von Gereral Electric). Weitere vorteilhafte Silikonelastomere können gewählt werden aus der Gruppe der Vinyl Dimethicone Crosspolymere, wie z. B. das Dow Corning 9506 Cosmetic Powder von Dow Corning (INCI: Dimethicone / Vinyl Dimethicone Crosspolymer).

Weiterhin vorteilhaft eingesetzt werden können sogenannte Silikonharze, wie z.B. KSP-100, KSP-200 oder KSP-300 von Shin Etsu, die ebenfalls unter der INCI bezeichnung Dimethicone /Vinyl Dimethicone Crosspolymer geführt werden oder SR 1000 von GE Bayer Silicones, das die INCI Bezeichnung Trimethylsiloxy Silicate trägt

Weiterhin bevorzugt ist auch Lauroyl Lysine, das unter der Bezeichnung Amihope LL von Ajinomoto vertrieben wird.

Die Gesamtmenge an mindestens einem Füllstoff in den fertigen kosmetischen oder dermatologischen Zubereitungen wird vorteilhaft aus dem Bereich von 0,05 - 20,0 Gew.-%, bevorzugt 0,5 - 10,0 Gew.-% gewählt, bezogen auf das Gesamtgewicht der Zubereitungen.

Erfindungsgemäß bevorzugte Füllstoffe kommen aus der Gruppe der anorganischen oder organischen Siliziumverbindungen.

Von den anorganischen Siliziumverbindungen sind besonders bevorzugt die Schichtsilikate. Davon besonders bevorzugt sind Kaolin, Talkum und Mica.

Weiterhin gehören zu den bevorzugten anorganischen Siliziumverbindungen die and der Oberfläche organisch modifizierten sphärischen Partikel.

Von diesen sind besonders bevorzugt die Polymethylsilsesquioxane und hydrophob modifizierte Aerosile, wie z.B. Aerosil R 972.

Zu den organischen Siliziumverbindungen gehören die Siloxan Elastomere und Siloxan Harze. Von denen sind besonders bevorzugt die KSP-Typen von Shin Etsu, sowie das Trimethylsiloxysilicate.

Weitere erfindungsgemäß bevorzugte Füllstoffe kommen aus der Gruppe der sphäreischen Partikel. Besonders bevorzugt ist der mittlere Teilchendurchmesser kleiner als 20 m. Weiterhin werden bevorzugt spärische Partikel organischen Ursprungs gewählt. Besonders bevorzugt ist davon das BPD-500, das von der Firma Kobo vertrieben wird.

Desweiteren sind bevorzugt sphärische Partikel mit einem mittlerern Teilchendurchmesser kleiner als 10 m. Davon besonders bevorzugt sind Nylon-12, das z.B. als SP-501 oder SP-500 von der Firma Kobo vertrieben wird. Weiterhin bevorzugt sind Polymethylmethacrylate, die z.B. unter dem Handelsnamen Covabead LH 85 von LCW vertrieben wird.

Weiterhin bevorzugt eingesetzt werden könnenWismut Oxychlorid oder Bornitrid.

Es ist erfindungsgemäß von Vorteil, wenn die Zubereitungen, dadurch gekennzeichnet sind, dass sie 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an einem oder mehreren Füllstoffen enthalten.

Verdicker werden erfindungsgemäß vorzugsweise gewählt aus der Gruppe der Hydrokolloide. "Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wässrigen Medien ist. Voraussetzung dafür ist, dass diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

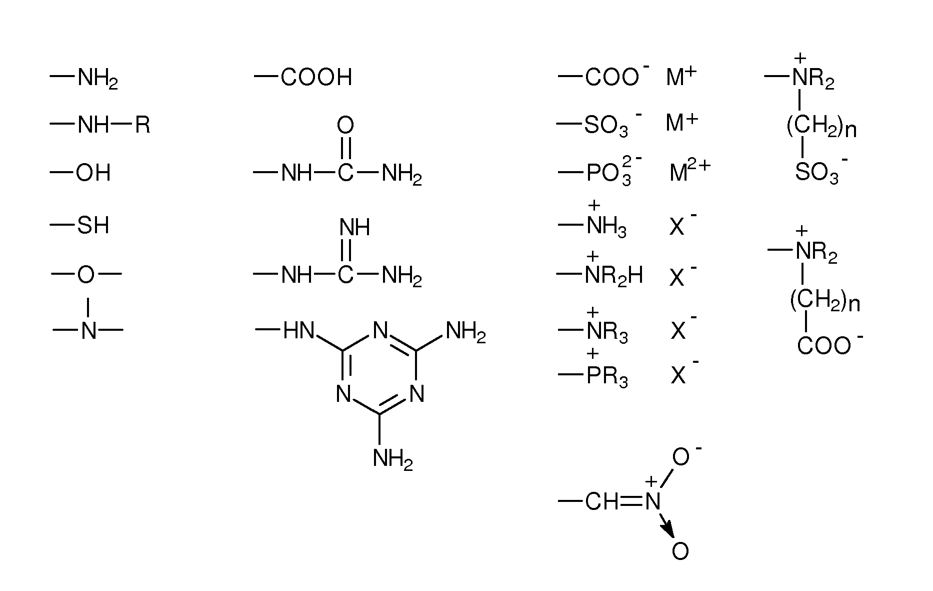

Die Gruppe der kosmetisch und dermatologisch relevanten Hydrokolloide lässt sich wie folgt einteilen in:
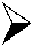 organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
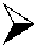 organische, abgewandelte Naturstoffe, wie z. B. Carboxymethylcellulose und andere Celluloseether, Hydroxyethyl- und -propylcellulose und dergleichen,
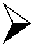 organische, vollsynthetische Verbindungen, wie z. B. Polyacryl- und Polymethacryl-Verbindungen, Vinylpolymere, Polycarbonsäuren, Polyether, Polyimine, Polyamide,
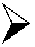 anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus

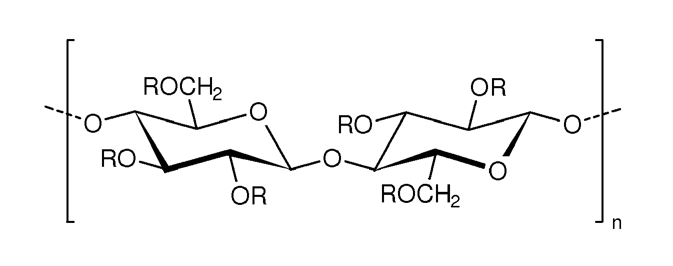

### Strukturformel I

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder CH₂-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2×10⁶ bis 24×10⁶ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 10⁶ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht.

Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlantischen, zu den Florideen zählenden Rotalgen (Chondrus crispus und Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht löslich ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt . Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute 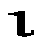 -Carrageenan ist sowohl wasserlöslich als auch gelbildend. Weitere Carrageen-Typen werden ebenfalls mit griechischen Buchstaben bezeichnet: α, β, γ, µ, ν, ξ, π, ω, χ. Auch die Art vorhandener Kationen (K⁺, NH₄⁺, Na⁺, Mg²⁺, Ca²⁺) beeinflusst die Löslichkeit der Carrageene.

Die Verwendung von Chitosan in kosmetischen Zubereitungen ist per se bekannt. Chitosan stellt ein partiell deacyliertes Chitin dar. Dieses Biopolymer hat u.a. filmbildende Eigenschaften und zeichnet sich durch ein seidiges Hautgefühl aus. Von Nachteil ist jedoch seine starke Klebrigkeit auf der Haut, die insbesondere - vorübergehend - während der Anwendung auftritt. Entsprechende Zubereitungen können dann im Einzelfalle nicht vermarktungsfähig sein, da sie vom Verbraucher nicht akzeptiert bzw. negativ beurteilt werden. Chitosan wird bekanntermaßen beispielsweise in der Haarpflege eingesetzt. Es eignet sich, besser als das ihm zugrunde liegende Chitin, als Verdicker oder Stabilisator und verbessert die Adhäsion und Wasserresistenz von polymeren Filmen. Stellvertretend für eine Vielzahl von Fundstellen des Standes der Technik: H.P.Fiedler, "Lexikon der Hilfsstoffe für Pharmazie, Kosmetik und angrenzende Gebiete", dritte Auflage 1989, Editio Cantor, Aulendorf, S. 293, Stichwort "Chitosan".

Chitosan ist gekennzeichnet durch folgende Strukturformel: 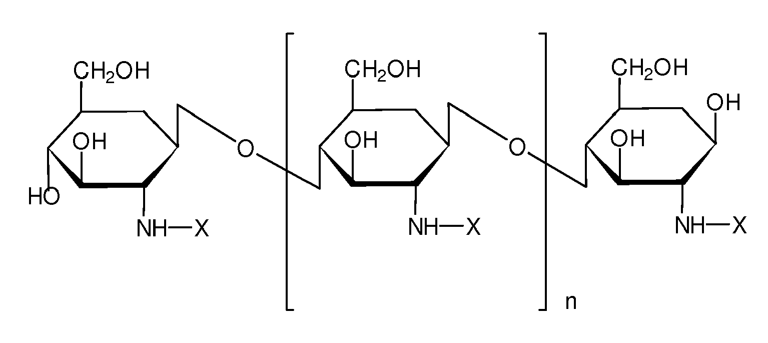 dabei nimmt n Werte bis zu ca. 10.000 an, X stellt entweder den Acetylrest oder Wasserstoff dar. Chitosan entsteht durch Deacetylierung und teilweise Depolymerisation (Hydrolyse) von Chitin, welches durch die Strukturformel 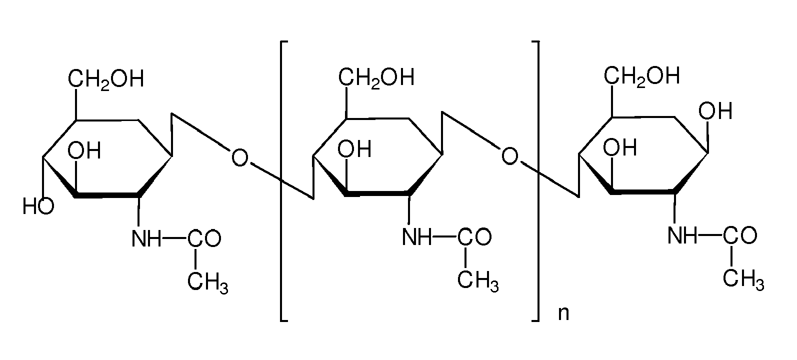 gekennzeichnet ist. Chitin ist wesentlicher Bestandteil des Ektoskeletts ['O χ 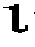 των = grch.: der Panzerrock] der Gliederfüßer (z.B. Insekten, Krebse, Spinnen) und wird auch in Stützgeweben anderer Organismen (z.B. Weichtiere, Algen, Pilze) gefunden.

Im Bereich von etwa pH <6 ist Chitosan positiv geladen und dort auch in wässrigen Systemen löslich. Es ist nicht kompatibel mit anionischen Rohstoffen. Daher bietet sich zur Herstellung chitosanhaltiger Öl-in-Wasser-Emulsionen der Einsatz nichtionischer Emulgatoren an. Diese sind an sich bekannt, beispielsweise aus der EP-A 776 657.

Erfindungsgemäß bevorzugt sind Chitosane mit einem Deacetylierungsgrad > 25 % , insbesondere > 55 bis 99 % [bestimmt mittels ¹H-NMR]).

Es ist von Vorteil, Chitosane mit Molekulargewichten zwischen 10.000 und 1.000.000 zu wählen, insbesondere solches mit Molekulargewichten zwischen 100.000 und 1.000.000. [bestimmt mittels Gelpermetionschromatographie].

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der so genannten Carbomere oder Carbopole (Carbopol® ist eigentlich eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

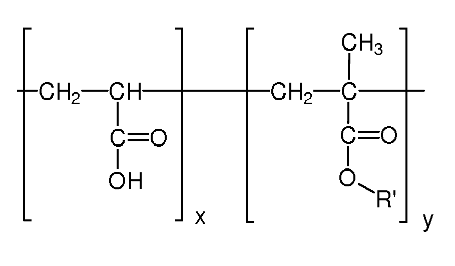

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind.

Ferner vorteilhaft sind Copolymere aus C₁₀₋₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen.

Die Gesamtmenge an einem oder mehreren Verdicker wird in den fertigen kosmetischen oder dermatologischen Zubereitungen vorteilhaft kleiner als 1,5 Gew.-%, bevorzugt zwischen 0,1 und 1,0 Gew.-%, bezogen auf das Gesamtgewicht der Zubereitungen, gewählt.

Vorteilhaft sind die erfindungsgemäßen Zubereitungen in Kombination mit nichtflüchtigen und flüchtigen Siliconölen (bevorzugt Dimethicone und Cyclomethicone) in einer Emulsion versehen. Dies hat den Vorteil, dass, dass die Folsäure länger auf der Haut verbleibt, die kosmetische Zubereitung sehr gut verteilbar ist und ein leichtes, angenehmes Hautgefühl aufweist.

Die Bevorzugte Ausführungsform enthält mehr flüchtiges Siliconöl (Cyclomethicone) als nichtflüchtiges Siliconöl (Dimethicone).

Erfindungsgemäß vorteilhaft sind Zubereitungen, dadurch gekennzeichnet, dass die Gewichtsverhältnisse von Folsäure und/oder deren Derivaten zu nichtflüchtigen Siliconölen zu flüchtigen Siliconölen (A : B : C) wie a : b : c gewählt wird, wobei a, b und c unabhängig voneinander rationale Zahlen von 1 bis 5, bevorzugt von 1 bis 3 darstellen.

Erfindungsgemäß besonders vorteilhaft sind Zubereitungen, dadurch gekennzeichnet, dass die Gewichtsverhältnisse von A : B : C zwischen 1:10:100 und 1:100:200, bevorzugt von etwa 1:50:150 gewählt werden.

Als nicht-flüchtig bezeichnet man ein Silikonöl, wenn es für mehrere Stunden auf der Haut oder den Lippen bleibt. Es hat einen Dampfdruck bei Raumtemperatur von kleiner als 0,02 mm Hg (2,66 Pa). Ein flüchtiges Silikonöl verdampft innerhalb von einer Stunde von der Haut oder den Lippen. Es hat einen Dampfdruck bei Raumtemperatur, der zwischen 0,02 mmHg und 300 mm Hg (13 Pa - 12000 Pa), bevorzugt zwischen 0,1 mmHg und 90 mm Hg (2,66 Pa - 40000 Pa) liegt.

Demgemäß sind erfindungsgemäß vorteilhaft Zubereitungen, dadurch gekennzeichnet, dass wenigstens ein Siliconöl mit einem Dampfdruck bei Raumtemperatur kleiner als 0,02 mm Hg (2,66 Pa) sowie mindestens ein Siliconöl mit einem Dampfdruck bei Raumtemperatur zwischen 0,02 mmHg und 300 mm Hg (13 Pa - 12000 Pa), gewählt werden.

Es wird bevorzugt, die Silicone der erfindungsgemäßen Zubereitungen aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

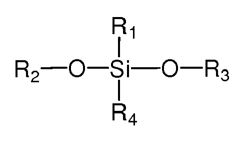

Als erfindungsgemäß vorteilhaft einzusetzenden linearen Silicone mit mehreren Siloxyleinheiten werden im allgemeinen durch Strukturelemente charakterisiert wie folgt: 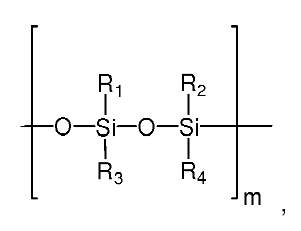 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende cyclische Silicone werden im allgemeinen durch Strukturelemente charakterisiert, wie folgt 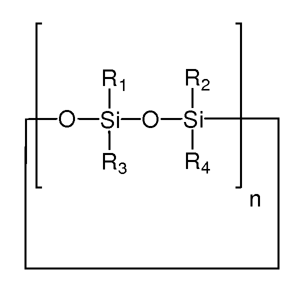 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). n kann dabei Werte von 3/2 bis 20 annehmen. Gebrochene Werte für n berücksichtigen, dass ungeradzahlige Anzahlen von Siloxylgruppen im Cyclus vorhanden sein können.

Vorteilhaft wird Phenyltrimethicon als Siliconöl gewählt. Auch andere Siliconöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Die kosmetischen oder dermatologischen Zubereitungen können erfindungsgemäß wie üblich zusammengesetzt sein und zur Behandlung, der Pflege und der Reinigung der Haut und/oder der Haare und als Schminkprodukt in der dekorativen Kosmetik dienen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindungsgemäß vorteilhaft, Folsäure und/oder deren Derivate in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, Folsäure und/oder deren Derivate in wässrige Systeme bzw. Tensidzubereitungen zur Reinigung der Haut und der Haare einzufügen.

Die bevorzugte Anwendung der erfindungsgemäßen Zubereitungen umfassend Folsäure und/oder deren Derivate sowie mindestens ein Parfumbestandteil, insbesondere gewählt aus der Gruppe Methylbenzoat, Limonen, Citral, Linalool, alpha-Isomethylionone, Geraniol, Methyl-2 Octynoat, Citronelol, Isoeugenol, Benzylbenzoat, Benzylalkohol und/oder Benzylsalicylat, bevorzugt Methylbenzoat, Citral, Limonen, Benzylbenzoat, Isoeugenol und/oder Citronelol, bevorzugt als eine Zweier- oder Dreierkombination dieser Stoffe, ist die Verwendung als Tagescreme, Nachtcreme, Augenpflegecreme, Handcreme, Fußcreme, Haarpflegeprodukt oder Bodyserum.

Als Kosmetikum, also keine therapeutische Verwendung, kann mit den erfindungsgemäßen Zubereitungen vorteilhaft Cellulite, Neurodermitis und/oder juckenden Hautzuständen behandelt werden.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Füllstoffe, Elektrolyte, organische Lösemittel oder Silikonderivate.

Die erfindungsgemäßen kosmetischen und dermatologischen Zubereitungen können vorteilhaft weitere Komplexbildner enthalten. Der oder die weiteren Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen, Phytinsäure, Natriumhexamethaphosphat, Ethylendiamindisuccinat, Nitrilotriessigsäure (NTA) und deren Anionen, Polyphenole aus der Gruppe der Flavonoide.

Der oder die weiteren Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Vorteilhaft umfasst die erfindungsgemäße Zubereitung UV-Filtersubstanzen, hierbei bevorzugt 0.1 Gew.-% bis 3.5 Gew.-%, besonders bevorzugt 0.5 Gew.-% bis 3 Gew.-%, insbesondere 1- 2 Gew.-%, bezogen auf das Gesamtgewicht der Zubereitungen, an UVA-Filtern aus der Gruppe der Triazine, insbesondere 2,4-Bis-([4-(2-ethyl-hexyloxy)-2-hydroxy]-phenyl}-6-(4-methoxyphenyl)-1,3,5-triazin (Tinosorb® S) oder 2,4-bis-[5-1 (dimethylpropyl)benzoxazol-2-yl-(4-phenyl)-imino]-6-(2-ethylhexyl)-imino-1,3,5-triazin (CAS Nr. 288254-16-0).

Ferner bevorzugt ist der Zusatz von 0.01 Gew.-% bis 0.4 Gew.-%, besonders bevorzugt 0.1 Gew.-% bis 0.4 Gew.-%, insbesondere 0.2 bis 0.3 Gew.-%, bezogen auf das Gesamtgewicht der Zubereitungen, an Ethylendiamintetraacetat.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze. Vorteilhafte UV-Filtersubstanzen im Sinne der vorliegenden Erfindung sind ferner so genannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- Dioctylbutylamidotriazon (INCI: Dioctylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)-ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wässrige Lösungen
- Öle, wie Triglyceride oder pflanzliche Öle, z.B. Caprylsäure/ Caprinsäuretriglyceride, Triisostearin, Avocadoöl, Kokosfettsäureglyceride, Macadamiaöl, Mandelöl, Jojobaöl, Rizinulsöl, Nachtkerzenöl oder Traubenkernöl
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; z.B. Mineralöl, Dibutyladipat, Pentaerythrityltetraisostearat, Isopropylmyristat, Isopropylpalmitat, Isopropylstearat, Dicaprylylcarbonat, Caprylylether, Alkylbenzoat, Cetearylisononanoat, Alkyltartrat, Alkyllaktat, Polydecen, Isohexadecan, Octyldeceylmyristat, Squalan, Octylcocoat, Octyldodecanol, Butylenglykolcaprylat/caprat, Dicaprylylcarbonat
- Silikonöle, wie z.B. Cyclomethicon, Dimethicon, Phenyltrimeticon
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Erfindungsgemäße als Emulsionen vorliegende Zubereitungen enthalten einen oder mehrere Emulgatoren. Diese Emulgatoren können vorteilhaft ausgewählt werden aus der Gruppe der nichtionischen, anionischen, kationischen oder amphoteren Emulgatoren. Beispiele sind u.a. PEG-40-Stearat , Sorbitanstearat, Glycerylstearatcitrat, Stearinsäure, oder Zuckerester, wie Polyglyceryl-3 Methylglucosedistearat:, Cetearylglucoside.

Weiterhin können zur Verbesserung des Hautgefühls Füllstoffe, wie z.B. Silica, Lauroyllysin, Polyethylen, Mica, Nylon oder Stärkederivate, wie z.B. Tapiokastärke, Aluminium Starch Octenylsuccinate, Distarchphosphat eingesetzt werden.

Erfindungsgemäß können weitere Antioxidantien und Wirkstoffe, wie z.B. Vitamin A und Derivate (Vitamin-A-Palmitat), Vitamin C und Derivate (Ascorbylalmitat, Mg-Ascorbylphosphat), Vitamin E und Derivate, ungesättigte Fettsäuren und Derivate (Linolsäure, Linolensäure, Ölsäure), alpha-Glucosylrutin, Ubiquinon (Q10), Harnstoff, Pflanzenextrakte, Ginko, Soja, Aloe Vera, Süßholz, Hamamelis, Bisabolol, Ginseng, Gurke, Biotin, Carotinoide, Taurin, Isoflavonoide, Licochalkon, Dydydroxyaceton, Aminosäuren, Zinkoxid, Panthenol, Dioic acid, enthalten sein.

Weiterhin können zur Verbesserung der Hautpflegeeigenschaften Moisturizer wie z.B. Glycerin, Butylenglykol, Propylen Glykol, Methylpropandiol, Ethylhexylglycerin, Sorbitol oder Harnstoff zugesetzt werden.

Zuberereitungen gemäß der vorliegenden Erfindung können auch Konservierungsmittel und Konservierungshelfer, wie z.B. Phenoxyethanol, Parabene, Benzylalkohol, Hexamidin Diisethionat, Jodopropyl Butylcarbamat, Diazolidinyl Urea, Hydantoin, Sorbinsäure und ihre Salze, Benzoesäure oder Methyldibromoglutaronitril enthalten.

Es folgen vorteilhafte Ausführungsbeispiele der vorliegenden Erfindung.

### Tagescreme

| Beispielrezeptur 1 | Gew.-% |
|---|---|
| Glycerylstearat | 2,6 |
| Stearylalkohol | 2 |
| PEG-40-Stearat | 0,8 |
| Weiße Vaseline | 3 |
| Cetylalkohol | 4 |
| Sheabutter | 3 |
| Titandioxid | 0,5 |
| Butylmethoxydibenzoylmethan | 2 |
| Lineares Silikonöl (Dimethicon) | 0,5 |
| Tapiokastärke | 1 |
| Methylparaben | 0,2 |
| Tocopherolacetat | 0,5 |
| Phenylbenzimidazolsulfonsäure Natrium-Salz | 1,5 |
| Ethlyhexylmethoxycinnamat | 7 |
| Phenoxyethanol | 0,4 |
| C₁₂₋₁₅ Alkylbenzoat | 3 |
| Propylparaben | 0,1 |
| Ethylparaben | 0,1 |
| Kreatin | 0,2 |
| Kreatinin | 0,05 |
| Folsäure | 0,03 |
| Glycerin | 8,7 |
| Polyacrylsäure, Na-Salz | 0,1 |
| Carrageenan | 0,2 |
| Ethylhexylglycerin | 0,5 |
| Na₃EDTA | 0,2 |
| Limonen | 0,0025 |
| Caprylic/Caprictriglycerid | 1 |
| Methylpropanediol | 4 |
| Wasser 100% | ad 100 |

### Nachtcreme

| Beispielrezeptur 2 | Gew.-% |
|---|---|
| Tapiokastärke | 2 |
| Cetylalkohol | 2 |
| Glycerylstearatcitrat | 2 |
| Sheabutter | 4 |
| Hydrierte Kokosglyceride | 3 |
| Ethlyhexylmethoxycinnamat | 5 |
| Phenoxyethanol | 0,4 |
| Kreatinin | 0,05 |
| Lineares Silikonöl (Dimethicon) | 1,5 |
| Zyklisches Silikonöl (Cyclomethicon) | 5 |
| Caprylylcarbonat | 5 |
| Nylon-12 | 1,5 |
| Tocopherylacetat | 1 |
| Squalan | 2 |
| Avocadoöl | 1 |
| Carrageenan | 0,1 |
| Polyacrylsäure, Na-Salz | 0,1 |
| Methylpropanediol | 4 |
| Kreatin | 0,2 |
| Methylparaben | 0,15 |
| Folsäure | 0,03 |
| Glycerin | 8,7 |
| Na₃EDTA | 0,2 |
| Methylbenzoat + Citral | 0,0004 |
| Butylenglykol + lodopropynylbutylcarbamat | 0,09 |
| C₁₂₋₁₅ Alkylbenzoat | 3 |
| Acrylates/C10-30 Alkyl Acrylate Crosspolymer | 0,1 |
| 1,2-Hexanediol | 0,5 |
| Wasser | ad 100 |

### Augenpflegecreme

| Beispielrezeptur 3 | Gew.-% |
|---|---|
| Glycerylstearat | 2,6 |
| Hydriertes Pflanzenöl | 1,5 |
| PEG-40-Stearat | 0,8 |
| Weiße Vaseline | 1,5 |
| Cetylstearylalkohol | 1,5 |
| Sheabutter | 3 |
| Titandioxid | 5 |
| Lineares Silikonöl (Dimethicon) | 2,5 |
| Tapiokastärke | 1,5 |
| Methylparaben | 0,21 |
| Tocopherolacetat | 0,5 |
| Nylon-12 | 1,5 |
| Phenoxyethanol | 0,4 |
| C₁₂₋₁₅ Alkylbenzoat | 2 |
| Zyklisches Silikonöl (Cyclomethicon) | 4 |
| Propylparaben | 0,05 |
| Ethylparaben | 0,11 |
| Methylpropanediol | 2 |
| Kreatin | 0,2 |
| Kreatinin | 0,05 |
| Octyldodecanol | 2 |
| Folsäure | 0,03 |
| Glycerin | 6,5 |
| Polyacrylsäure, Na-Salz | 0,1 |
| Caprylylglycol | 0,25 |
| Na₃EDTA | 0,2 |
| Benzyl Benzoat | 0,0025 |
| Caprylic/Caprictriglycerid | 2 |
| Wasser 100% | ad 100 |

### Bodyserum

| Beispielrezeptur 4 | Gew.-% |
|---|---|
| Glycerylstearat | 2 |
| Squalan | 1,5 |
| PEG-40-Stearat | 1 |
| Medizinisches Weißöle (Gemisch verschiedener Viskositäten) | 15,1 |
| Stearylalkohol | 3,5 |
| Hydrierte Kokosglyceride | 3 |
| Lineares Silikonöl (Dimethicon) | 0,5 |
| Myristylmyristat | 1,5 |
| Methylparaben | 0,3 |
| Tocopherolacetat | 0,5 |
| Nylon-12 | 1,5 |
| Phenoxyethanol | 0,5 |
| C₁₂₋₁₅ Alkylbenzoat | 2 |
| Zyklisches Silikonöl (Cyclomethicon) | 4 |
| Propylparaben | 0,1 |
| Ethylparaben | 0,11 |
| Methylpropanediol | 2 |
| Kreatin | 0,5 |
| Kreatinin | 0,01 |
| Octyldodecanol | 2 |
| Butylmethoxydibenzoylmethan | 0,5 |
| Octocrylene | 6 |
| Folsäure | 0,003 |
| Glycerin | 10 |
| Polyacrylsäure, Na-Salz | 0,02 |
| Caprylylglycol | 0,25 |
| Na₃EDTA | 0,2 |
| Isoeugenol + Citronelol | 0,0035 |
| Caprylic/Caprictriglycerid | 2 |
| Wasser 100% | ad 100 |

### O/W-Emulsion

| Beispielrezeptur 5 | Gew.-% |
|---|---|
| Caprylsäure/Caprinsäuretriglyceride | 3 |
| Sorbitanstearat | 1 |
| Stearylalkohol | 1 |
| Polyglyceryl-3 Methylglucosedistearat: | 3 |
| 2,4-bis-[5-1 (dimethylpropyl)benzoxazol-2-yl-(4-phenyl)-imino]-6-(2-ethylhexyl)-imino-1,3,5-triazin (CAS Nr. 288254-16-0) | 1 |
| Butyl Methoxydibenzoylmethane | 1 |
| Ethlyhexylmethoxycinnamat | 3 |
| Ethylhexyltriazon | 1 |
| Lineares Silikonöl (Dimethicon) | 2 |
| Zyklisches Silikonöl (Cyclomethicon) | 4 |
| C₁₂₋₁₅ Alkylbenzoat | 2 |
| Dicaprylylether | 2 |
| Carrageenan | 0,1 |
| Vernetztes Alkylacrylat (Alkylacrylate Crosspolymer) | 0,1 |
| Kreatin | 0,4 |
| Folsäure | 0,05 |
| Glycerin | 8 |
| Methyl-2 Octynoat | 0,001 |
| Na₃EDTA | 0,2 |
| Licochalkon A | 0,02 |
| Ethylhexylglycerin | 0,4 |
| Citronensäure, Natriumsalz (pH 6.5) | q.s. |
| Wasser | ad 100 |

### Sonnenschutzcreme

| Beispielrezeptur 6 | Gew.-% |
|---|---|
| Glycerylstearat | 2,5 |
| PEG-40-Stearat | 1 |
| Cetylalkohol | 2,5 |
| C₁₂₋₁₅ Alkylbenzoat | 2 |
| Hydrierte Kokosfettglyceride | 2 |
| Octyldodecanol | 2 |
| Zyklisches Silikonöl (Cyclomethicon) | 2 |
| Bienenwachs | 1 |
| Ethylhexylmethoxycinnamat | 6 |
| Phenylbenzimidazolsulfonsäure | 2 |
| Natriumascorbylphosphat | 0,1 |
| Methylpropandiol | 2 |
| Diazolidinylharnstoff | 0,1 |
| Carrageenan | 0,1 |
| Polyacrylsäure, Na-Salz | 0,1 |
| Isoeugenol + Benzylbenzoat | 0,2 |
| Glycerin | 7 |
| Folsäure | 0,03 |
| Na₃EDTA | 0,2 |
| 2,4-Bis-{[4-(2-ethyl-hexyloxy)-2-hydroxy]-phenyl}-6-(4-methoxyphenyl)-1,3,5-triazin (Tinosorb® S) | 2 |
| Natronlauge (pH 6.8) | q.s. |
| Wasser | ad 100 |

### Beispiel 7 - 10

| O/W - Emulsionen | 7 | 8 | 9 | 10 |
|---|---|---|---|---|
| Parfum | 0,3 | | 0,3 | |
| Na₃HEDTA | 0,5 | 1 | 0,5 | 1 |
| Glycerin | 10 | 7,5 | 7,5 | 25 |
| Glycerylstearatcitrat | 2 | 2,5 | | |
| PEG-40 Stearat | | | 1,0 | 0,7 |
| Glycerylstearat SE | | | 2,5 | 2,5 |
| Stearylalkohol | 2 | 3 | 1 | 1,5 |
| Cetylalkohol | 2 | | 1 | |
| Polyacrylsäure, Na-Salz | 0,1 | 0,25 | 0,3 | 0,1 |
| Acrylate/C10-30 Alkylacrylat Crosspolymer | 0,2 | 0,3 | 0,1 | 0,2 |
| Chondrus Crispus | 0,2 | | | |
| Myristylmyristat | | | 1 | 1 |
| C₁₂₋₁₅ Alkylbenzoat | 2 | 4 | 2 | 4 |
| Octyldodecanol | 2 | | | 2 |
| Caprylsäure/Caprinsäuretriglyceride | 1 | 2 | 1 | 2 |
| Medizinisches Weißöl | 3 | | 2 | |
| Butyrospermum Parkii | | 3 | 3 | |
| Lineares Silikonöl (Dimethicon) | 1 | 2 | 1 | 3 |
| Zyklisches Silikonöl (Cyclomethicon) | 3 | 4 | 1,6 | 3 |
| Methylparaben | 0,4 | 0,3 | | 0,2 |
| Phenoxyethanol | 0,4 | 0,6 | 0,4 | 0,5 |
| Diazolidinylharnstoff | | | 0,2 | |
| Ethylhexylglycerin | 0,3 | | | |
| Caprylglycol | 0,25 | | | |
| 1,2-Hexandiol | | 0,5 | | 0,5 |
| Methylpropandiol | 3 | | 2 | 2 |
| Butylmethoxydibenzoylmethan | | 2 | | 3 |
| Ethylhexylmethoxycinnamat | | 5 | | 7 |
| Phenylbenzimidazolsulfonsäure | | 2 | | 2 |
| Nylon-12 | | 1,5 | 1,5 | |
| Titandioxid hydrophil gecoated | | | 5 | |
| Titandioxid hydrophob gecoated | 0,5 | 2 | 1,5 | 0,5 |
| Tapiocastärke | 2 | | 1,5 | 3 |
| Talkum | 1,5 | 2 | 0,5 | |
| Tocopherolacetat | 0,5 | 1 | 1 | 1,5 |
| Citral + alpha-Isomethyl lonone + Benzylsalicylat | 0,002 | 0,01 | 0,25 | 0,0035 |
| Folsäure | 0,05 | 0,1 | 0,025 | 0,1 |
| Kreatin | 0,2 | 0,5 | 0,2 | 0,1 |
| Kreatinin | 0,2 | 0,1 | 0,05 | 0,1 |
| Wasser | Ad 100 | Ad 100 | Ad 100 | Ad 100 |

## Patentansprüche

1. Kosmetische oder dermatologische Zubereitungen mit einem Gehalt an Folsäure und/oder deren Derivaten umfassend mindestens ein Parfumbestandteil gewählt aus der Gruppe 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, alpha-Isomethylionon, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citral, Citronellol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Geraniol, Guajakholzöl, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linaylacetat, Mandarinenöl, Menthyl PCA, Methyl 2-Octynoat, Methylbenzoat, Methylheptenon, Muskatnussöl, Rosmarinöl, süßes Orangenöl, Terpineol, Tonkabohnenöl, Triethylcitrat und/oder Vanillin.

2. Kosmetische oder dermatologische Zubereitungen nach Anspruch 1, **dadurch gekennzeichnet, dass** als Parfumbestandteil mindestens ein Bestandteil aus der Gruppe Methylbenzoat, Limonen, Citral, Linalool, alpha-Isomethylionone, Geraniol, Methyl-2 Octynoat, Citronelol, Isoeugenol, Benzylbenzoat, Benzylalkohol und/oder Benzylsalicylat gewählt werden.

3. Kosmetische oder dermatologische Zubereitungen nach Anspruch 1, **dadurch gekennzeichnet, dass** als Parfumbestandteil mindestens ein Bestandteil aus der Gruppe Methylbenzoat, Citral, Limonen, Benzylbenzoat, Isoeugenol und/oder Citronelol gewählt wird

4. Kosmetische oder dermatologische Zubereitungen nach Anspruch 1, 2 oder 3, **dadurch gekennzeichnet, dass** aus den Parfumbestandteilen zwei, drei, vier oder fünf Bestandteile ausgewählt werden.

5. Kosmetische oder dermatologische Zubereitungen nach einem der vorstehenden Ansprüche, **dadurch gekennzeichnet, dass** sie 0,001 Gew.-% bis 0,5 Gew.-%, besonders bevorzugt 0,005 Gew.-% bis 0,2 Gew.-%, insbesondere 0,01 bis 0,1 Gew.-%, bezogen auf das Gesamtgewicht der Zubereitungen, an Folsäure und/oder deren Derivaten enthalten.

6. Kosmetische oder dermatologische Zubereitungen nach einem der vorstehenden Ansprüche, **dadurch gekennzeichnet, dass** der Anteil an einem oder mehreren Parfumbestandteilen mindestens 0,00001 Gew.-%, bevorzugt mindestens 0,001 Gew.-%, bezogen auf die Gesamtmasse der Zubereitung, beträgt.

7. Kosmetische oder dermatologische Zubereitungen nach einem der vorstehenden Ansprüche, **dadurch gekennzeichnet, dass** der Anteil an einem oder mehreren Parfumbestandteilen maximal 1 Gew.-%, bevorzugt maximal 0,4 Gew.-%, bezogen auf die Gesamtmasse der Zubereitung, beträgt.

8. Kosmetische oder dermatologische Zubereitungen nach einem der vorstehenden Ansprüche umfassend ein oder mehrere Polyole, insbesondere Polyole, die 2 bis 3 Sauerstoffatome und mindestens 4 Kohlenstoffatome enthalten.

9. Kosmetische oder dermatologische Zubereitungen nach einem der vorstehenden Ansprüche umfassend ein oder mehrere Füllstoffe und/oder Verdicker.

10. Kosmetische oder dermatologische Zubereitungen nach einem der vorstehenden Ansprüche umfassend nichtflüchtige und/oder flüchtigen Siliconölen, insbesondere Dimethicone und/oder Cyclomethicone.

11. Kosmetische oder dermatologische Zubereitungen nach Anspruch 10, **dadurch gekennzeichnet, dass** die Gewichtsverhältnisse von Folsäure und/oder deren Derivaten (A) zu nichtflüchtigen Siliconölen (B) zu flüchtigen Siliconölen (C) wie a : b : c gewählt wird, wobei a, b und c unabhängig voneinander rationale Zahlen von 1 bis 5, bevorzugt von 1 bis 3 darstellen

12. Verwendung von mindestens einem Parfumbestandteile gewählt aus der Gruppe 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, alpha-Isomethylionon, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citral, Citronellol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Geraniol, Guajakholzöl, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linaylacetat, Mandarinenöl, Menthyl PCA, Methyl 2-Octynoat, Methylbenzoat, Methylheptenon, Muskatnussöl, Rosmarinöl, süßes Orangenöl, Terpineol, Tonkabohnenöl, Triethylcitrat und/oder Vanillin zur Verbesserung der olfaktorische Eigenschaften Folsäure-haltiger und/oder deren Derivate enthaltende kosmetische und/oder dermatologische Zubereitungen.

13. Verwendung von mindestens einem Parfumbestandteile gemäß Anspruch 12 zur Stabilisierung von Folsäure und/oder deren Derivate in kosmetischen und/oder dermatologischen Zubereitungen.

14. Verwendung der Zubereitungen nach einem der vorstehenden Ansprüche als Tagescreme, Nachtcreme, Augenpflegecreme, Handcreme, Fußcreme, Haarpflegeprodukt oder Bodyserum.

15. Verwendung der Zubereitungen nach einem der vorstehenden Ansprüche zur nichttherapeutischen Behandlung von Cellulite, Neurodermitis und/oder juckenden Hautzuständen.
